# EUROPEAN PATENT APPLICATION

(11) **EP 3 101 421 A1**
(43) Date of publication of application: **07.12.2016**
(21) Application number: 15743933.2
(22) Date of filing: 28.01.2015
(51) Int. Cl.: G01N 33/483, G01N 21/64

(54) **CELL MEMBRANE OBSERVATION AND ANALYSIS DEVICE AND CELL MEMBRANE OBSERVATION AND ANALYSIS METHOD**

(30) Priority: 30.01.2014 JP 2014015080
(71) Applicant: Namiki Seimitsu Houseki kabushikikaisha, Adachi-ku Tokyo 123-8511 (JP); National University Corporation Yamagata University, Yamagata-shi, Yamagata 990-8560 (JP)
(72) Inventor: NAKAYA Takayuki, Yuzawa-shi Akita 012-0855 (JP); SAKANE Rebun, Yuzawa-shi Akita 012-0855 (JP); ABE Kiyoto, Yuzawa-shi Akita 012-0855 (JP); WADA Yuya, Yuzawa-shi Akita 012-0855 (JP); OKUNO Takashi, Yamagata-shi Yamagata 990-8560 (JP); SATO Keishi, Yamagata-shi Yamagata 990-8560 (JP)
(74) Representative: Prinz & Partner mbB
(86) International application number: PCT/JP2015/052264
(87) International publication number: WO 2015/115448

(57) **Abstract**

A cell membrane observation and analysis device includes at least: a microscope; and a substrate which has a plurality of holes, in which a cell membrane is placed on each hole of the substrate to be placed on the substrate, the cell membrane is immersed into a buffer solution while the cell membrane is suctioned through the hole, and the buffer solution is sealed, thereby observing and analyzing the cell membrane. Using the above-mentioned device, a substrate is prepared; a cell membrane is dispersed in a buffer solution, thereby preparing a suspension; the suspension is dripped onto a face of the substrate; the cell membrane is sucked through each hole to be placed on each hole; the cell membrane is immersed into a second buffer solution while the cell membrane is sucked; the second buffer solution is sealed; and the cell membrane is observed and analyzed with a microscope.

## Description

### Technical Field

The present invention relates to a cell membrane observation and analysis device and a cell membrane observation and analysis method capable of observing or analyzing a vital function of a cell membrane from both faces of a cell membrane.

### Background Art

Anti-cancer drugs have strong side effects compared to general drugs and patients have different effective drugs. Thus, there is a case where an enough medical treatment effect cannot be obtained depending on the patient. Further, if a medical treatment effect cannot be obtained although expensive anti-cancer drugs were administered to the patient, medical expenses consequently increases. In order to solve these problems, there has been proposed an anti-cancer drug sensitivity test of examining whether the anti-cancer drug is effective for each patient before the anti-cancer drugs are administered to the patients.

As a method of the drug sensitivity test for the cell having been known so far, there is known a method in which a cancer cell obtained from a sampled piece cut during surgery or biopsy is prepared as a cell suspension through a cell culture medium reflux method or an enzymatic treatment method, the cell suspension is seeded onto a culture plate together with a culture solution including anti-cancer drugs, the result is incubated for several days to several tens of days, and the result is dyed by Giemsa stain or illuminated by light so as to examine whether the cancer cell has sensitivity with respect to the used anti-cancer drugs (for example, see Patent Literature 1).

Patent Literature 1: Japanese Examined Patent Application Publication No. 06-090205

### DISCLOSURE OF THE INVENTION

### Technical Problem

However, a vital function necessary to analyze the sensitivity of the drugs occurs in the cell membrane. Thus, there is a need to highly sensitively observe and analyze the vital functions relating to the transportation of drugs (a pump function of receiving or discharging drugs), infections, and the delivery of information occurring in the cell membrane in order to highly reliably perform the drug sensitivity test. However, since a test for the cell is performed in the drug sensitivity test of the related art, it is difficult to highly sensitively track the vital function of the cell membrane. Further, a large number of cell samples are needed in the drug sensitivity test and some time is inevitably taken for the test.

The present invention is made in consideration of the above-described circumstances and an object of the invention is to provide a cell membrane observation and analysis device and a cell membrane observation and analysis method capable of highly sensitively tracking a vital function occurring in a cell membrane.

### Technical Solution

The above-described objects are achieved by the following invention. That is, a cell membrane observation and analysis device according to the invention includes at least a microscope and a substrate which has a plurality of holes, wherein a cell membrane is placed on each hole of the substrate so that the cell membrane is placed on the substrate, the cell membrane is immersed into a buffer solution while the cell membrane is suctioned through the hole, and the buffer solution is sealed, thereby observing and analyzing the cell membrane.

An embodiment of the cell membrane observation and analysis device according to the invention further includes a substrate placing member that places the substrate thereon, wherein abutting portions of two orthogonal axial directions may be provided in at least a part of an outermost face of the substrate placing member and in a substrate placement portion for placing the substrate in parallel with two axes.

Another embodiment of the cell membrane observation and analysis device according to the invention further includes a light transmission window through which light is transmitted and which is provided at the other side of a substrate placement side in the substrate placing member, wherein the cell membrane may be irradiated with light transmitted through the light transmission window, and wherein the light transmission window may be detachable from the substrate placing member.

Furthermore, in another embodiment of the cell membrane observation and analysis device according to the invention, a vital function of each of faces of the cell membrane may be individually analyzed in each face.

Furthermore, in another embodiment of the cell membrane observation and analysis device according to the invention, the light transmission window may be formed of glass having light transmissivity of 90% or more with respect to visible light.

A cell membrane observation and analysis method of the invention includes: preparing a substrate having a plurality of holes; preparing a suspension by dispersing a cell membrane in a buffer solution; dripping the suspension onto a face of the substrate; suctioning the cell membrane through each hole by the pump so that the cell membrane is placed on each hole; immersing the cell membrane into a second buffer solution while continuously suctioning the cell membrane; sealing the second buffer solution; and observing and analyzing the cell membrane by a microscope.

An embodiment of the cell membrane observation and analysis method according to the invention further includes: preparing a substrate placing member having a substrate placed thereon, the substrate placing member having abutting portions of two orthogonal axial directions provided in at least a part of an outermost face of the substrate placing member and in a substrate placement portion for placing the substrate in parallel with two axes; bringing two orthogonal edges of the outermost face of the substrate into contact with the abutting portions of the substrate placement portion so that the substrate is placed on the substrate placement portion of the substrate placing member; and bringing the abutting portions of two orthogonal axial directions of the outermost face of the substrate placing member into contact with abutting unit of two orthogonal axial directions in a substrate placing member placement position of the microscope so that the substrate placing member is placed on the substrate placing member placement position of the microscope.

Furthermore, in another embodiment of the cell membrane observation and analysis method according to the invention, a vital function of each of faces of the cell membrane may be analyzed in each face.

### Advantageous Effects

According to the cell membrane observation and analysis device of the invention, the cell membrane can be observed and analyzed from both faces. Thus, since it is possible to highly sensitively track the vital function occurring in the cell membrane, there is no need to prepare a large number of cell samples for the drug sensitivity test, and thus the test can be conducted rapidly. Moreover, the effort of exchanging the samples is saved and both faces of the cell membrane can be observed and analyzed rapidly.

### Brief Description of the Drawings

Fig. 1A is a perspective view schematically showing a substrate included in a cell membrane observation and analysis device of an embodiment of the invention.
Fig. 1B is an enlarged top view schematically showing a plurality of holes formed in the substrate shown in Fig. 1A.
Fig. 2A is an enlarged cross-sectional view schematically showing the plurality of holes formed in the substrate included in the cell membrane observation and analysis device of the embodiment of the invention and a cell membrane placed on each hole.
Fig. 2B is an enlarged cross-sectional view schematically showing a state where a part of the cell membrane shown in Fig. 2A is removed and a part of the remaining cell membrane is placed on each hole of the substrate.
Fig. 3 is an enlarged cross-sectional view schematically showing the cell membranes placed on the plurality of holes formed in the substrate included in the cell membrane observation and analysis device of the embodiment of the invention and a state where the cell membranes are suctioned.
Fig. 4A is a top view of a substrate placing member included in the cell membrane observation and analysis device of the embodiment of the invention.
Fig. 4B is a front view of the substrate placing member included in the cell membrane observation and analysis device of the embodiment of the invention.
Fig. 4C is a bottom view of the substrate placing member included in the cell membrane observation and analysis device of the embodiment of the invention.
Fig. 5A is a top view showing the substrate placing member included in the cell membrane observation and analysis device of the embodiment of the invention and a fixed state of a saucer.
Fig. 5B is a front view showing the substrate placing member included in the cell membrane observation and analysis device of the embodiment of the invention, an inner structure thereof, and a fixed state of the substrate and the saucer.
Fig. 5C is a bottom view showing the substrate placing member included in the cell membrane observation and analysis device of the embodiment of the invention and a fixed state of the saucer.
Fig. 6A is an enlarged perspective view schematically showing a state where the substrate is placed on the substrate placing member included in the cell membrane observation and analysis device of the embodiment of the invention.
Fig. 6B is an enlarged perspective view schematically showing a state where the substrate is placed on the substrate placing member shown in Fig. 6A.
Fig. 7 is a cross-sectional view showing a state where a light transmission window, a window restraining member, the substrate placing member, the substrate, and the saucer are assembled.
Fig. 8 is a cross-sectional view showing a state where the light transmission window, the window restraining member, the substrate placing member, the substrate, and the saucer have been assembled.
Fig. 9A is a perspective view schematically showing a state where the substrate placing member is placed in the cell membrane observation and analysis device of the embodiment of the invention.
Fig. 9B is a perspective view schematically showing a state where the substrate placing member has been placed in the cell membrane observation and analysis device of the embodiment of the invention.
Fig. 10 is an explanatory diagram schematically showing a state where the substrate placed on the substrate placing member shown in Fig. 8 is observed and analyzed by an inverted microscope.
Fig. 11 is an explanatory diagram schematically showing a state where the substrate placed on the substrate placing member shown in Fig. 8 is observed and analyzed by an upright microscope.
Fig. 12 is a fluorescence microscope picture of a HeLa cell membrane captured and placed on each of holes formed in a substrate which is included in a cell membrane observation and analysis device of an example according to the invention.

### BEST MODE FOR CARRYING OUR THE INVENTION

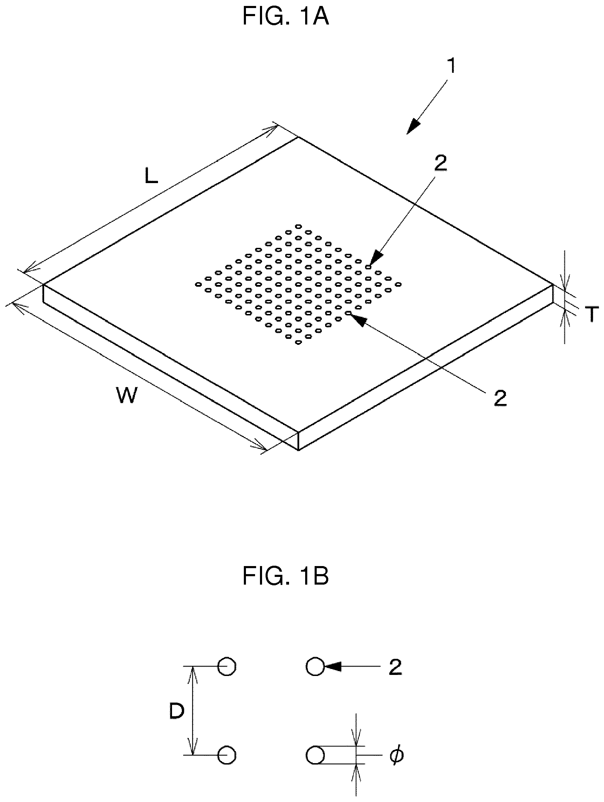

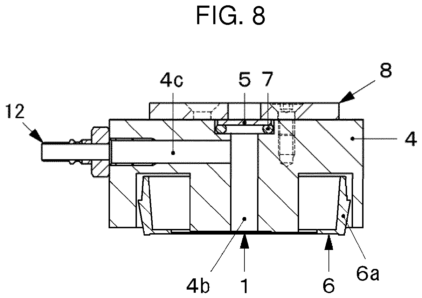

Hereinafter, a cell membrane observation and analysis device and a cell membrane observation and analysis method according to an embodiment of the invention will be described in detail with reference to Figs. 1 to 11. The cell membrane observation and analysis device of the embodiment includes a substrate having a cell membrane placed thereon and observes and analyzes the cell membrane placed on the substrate.

As a substrate 1, a substrate 1 having an external dimension in which a length L is 10 mm, a width W is 10 mm, and a thickness T is 0.2 mm and having a plurality of holes 2 formed therein as shown in Fig. 1A is prepared. The substrate may be formed of a material of a light transmitting transparent body or materials other than the transparent body. The material of the transparent body is, for example, quartz glass or sapphire, and the quartz glass has a property in which light transmissivity is 90% or more and a refractive index is 1.5 with respect to visible light having a wavelength λ of 588 nm. Meanwhile, the sapphire has a property in which light transmissivity is 80% or more and a refractive index is 1.8 with respect to visible light having a wavelength λ of 588 nm. Further, the substrate which is formed of materials other than the transparent body may be a substrate which is formed of plastic or silicon having no light-transmissivity with respect to a visible light region or a substrate having no light-transmissivity by attaching a shielding film or a seal to a front face of the substrate 1. Here, the holes 2 are formed on the shielding film or the seal attached to the substrate.

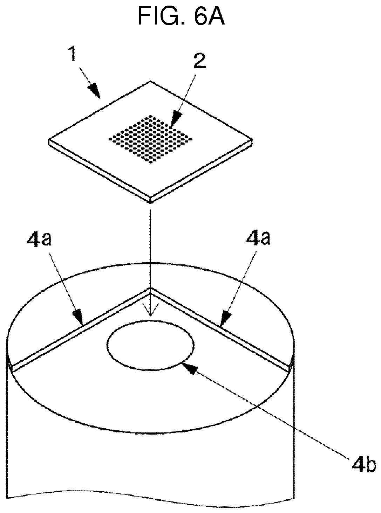

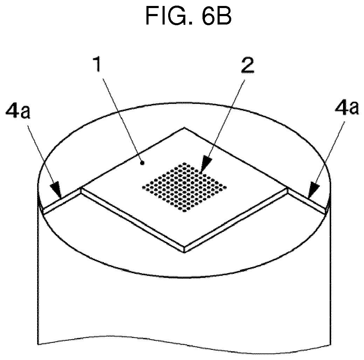

From Fig. 1B, the planar shape of the hole 2 is circular and the cross-sectional shape of the hole 2 is formed as a straight hole shape or a tapered hole shape although not shown in the drawings. When the hole 2 is formed in a tapered cross-sectional shape, a small diameter is formed at a side on which the cell membrane is placed. 500 or more holes are required as the number of hole samples minimally necessary to observe and analyze the vital function of the cell membrane and a total of 625 holes are formed as 25 units by 25 lines. A range in which each hole 2 is formed is set to be within 1 mmsquare. The diameter φ of each hole 2 is set to 2.0 μm, the gap D between the center portions of the holes 2 is set to 40 μm, and the aspect ratio of each hole 2 is set to 100 (a value obtained by dividing the substrate thickness T of 0.2 mm by the hole diameter φ of 2.0 μm). In Fig. 1A or Fig. 6, from the viewpoint of the easy comprehension of the drawings, a part of the holes 2 is omitted and the ratio of the diameter φ of each hole 2 with respect to the external dimension of the substrate 1 is magnified.

As the cell membrane, a cell membrane derived from a cancer cell of cells such as a human cell, a mammalian cell other than a human cell, or an animal cell like an insect cell is set as an observation and analysis target. Further, the incubated cell membrane may be set as a target sample. As a sample including the cell membrane of the cell, a sample including at least 500 to 100,000 cell membranes is desirable.

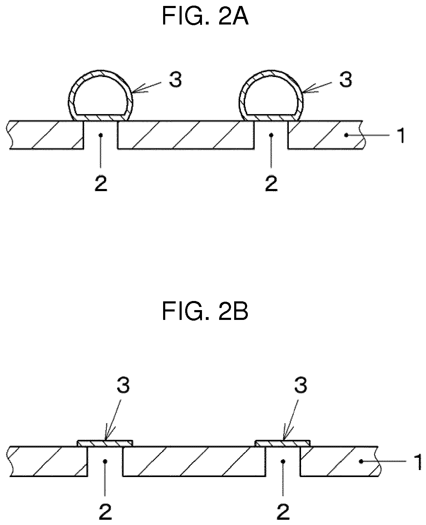

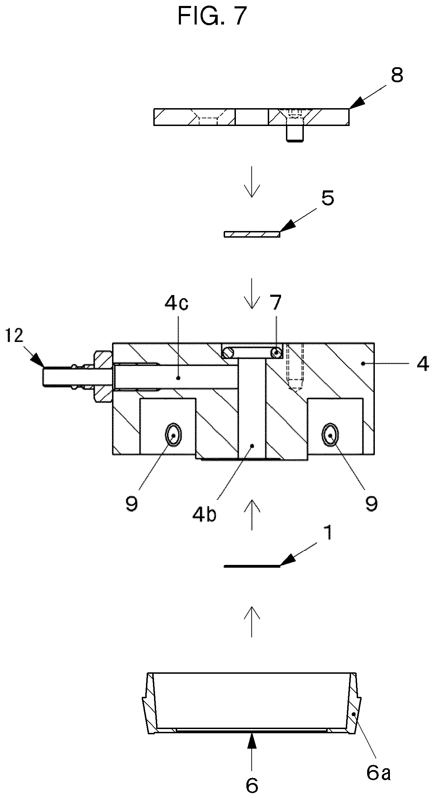

By dispersing the cell membrane in a buffer solution and dripping a suspension thereof onto the face of the substrate 1, the cell membrane 3 is adsorbed to the face of the substrate 1 in a round shape as shown in Fig. 2A. A predetermined amount of second buffer solution is charged into a saucer shown in Fig. 7. As the buffer solution, PBS (Phosphate Buffered Saline) may be used as an example, but the invention is not limited thereto. The cell membrane may be cleaned by the buffer solution.

The cell membrane 3 adsorbed to the face of the substrate 1 in a round shape is subjected to physical stimuli such as ultrasonic waves so that the rounded portion of the cell membrane 3 is removed and only a part of the cell membrane which is adsorbed to the face of the substrate 1 in advance is left as shown in Fig. 2B. When a shielding film or a seal is attached to the front face of the substrate 1, the front face of the substrate 1 onto which the cell membrane 3 is adsorbed is set as a face opposite to a face to which the shielding film or the seal is attached.

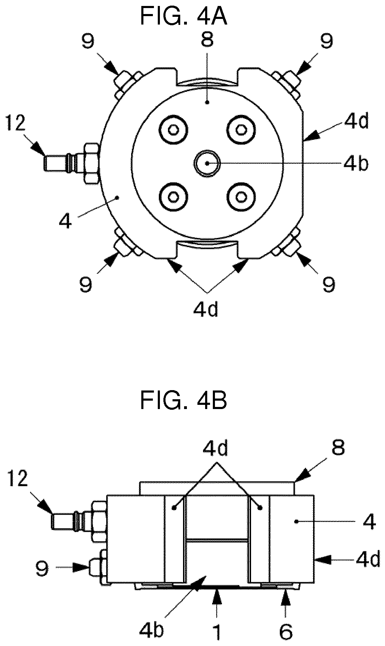

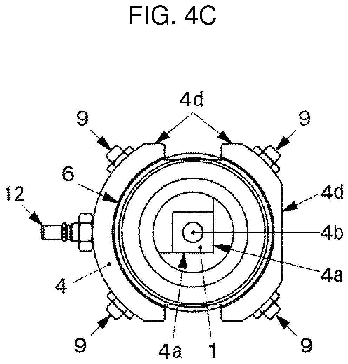

Next, the substrate 1 having a part of the cell membrane 3 adsorbed to the face thereof is placed on a substrate placing member 4 as shown in Figs. 4 to 8. The substrate placing member 4 is formed of stainless steel and is formed to include a light transmission window placement portion for placing a light transmission window 5, a substrate placement portion for placing the substrate 1, and a spot-facing portion which is formed in the circumferential direction to receive a peripheral edge portion 6a of the saucer 6.

In the substrate placing member 4, the light transmission window placement portion is provided at the substrate placement portion side. The light transmission window is supported by a ring member 7 of which the planar shape is formed in an O-shape and is placed inside the light transmission window placement portion. Further, the top face thereof is restrained and fixed by a restraining member 8 for the light transmission window 5. In this way, the light transmission window is included in the substrate placing member 4. It is desirable that the light transmission window 5 be formed of glass having a property in which light transmissivity is 90% or more and a refractive index is 1.5 with respect to visible light. As the wavelength of the visible light, 588 nm can be exemplified. Further, the restraining member 8 is formed of, for example, PTFE (polytetrafluoroethylene) and four corners thereof are fixed to one face side of the substrate placing member 4 by bolts-fastening. In this way, the light transmission window 5 is provided to be detachable from the substrate placing member 4.

One face of the substrate placing member 4 is provided with abutting portions 4a and 4a which are provided in the substrate placement portion for placing the substrate 1 and are provided in directions parallel to two orthogonal axes as shown in Figs. 4 to 8. The height of the abutting portions 4a and 4a is set to be equal to or higher than the thickness T of the substrate. As shown in Figs. 4 to 8 (particularly, Fig. 6), two orthogonal edges of the outermost face of the substrate 1 having the cell membrane dripped thereto contact the abutting portions 4a and 4a of two orthogonal axes directions, which are formed at one face of the substrate placing member 4, so that the substrate 1 is placed on the substrate placing member 4. At this time, the external dimension of the substrate 1 and the formation position of the hole 2 are set in advance so that all holes 2 are placed on the lighting holes 4b formed inside the substrate placing member 4 .

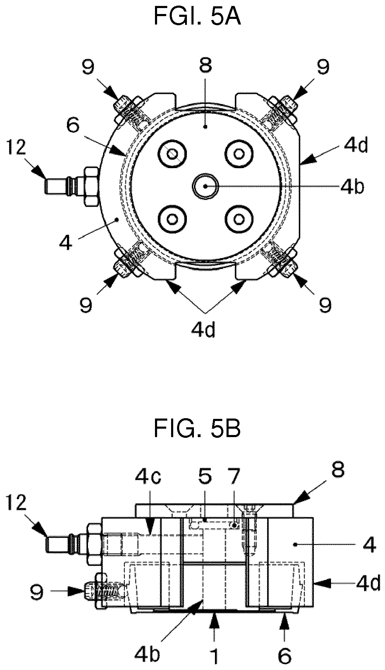

The hole 4b is formed in a circular shape having a diameter of about 5.0 mm and is formed in the substrate placing member 4 to connect between the substrate placement portion for placing the substrate 1 and the light transmission window placement portion for placing the light transmission window 5 as shown in Figs. 5B, 7, and 8. Thus, the cell membrane 3 dripped onto the face of the substrate 1 is irradiated with the light transmitted through the light transmission window 5. Moreover, a second hole 4c is formed from a side face of the hole 4b in a direction orthogonal to the hole 4b and is connected to a pump such as a vacuum pump (not shown) through a joint port (opening) 12. The hole 4c is also formed in a circular shape having a diameter of about 5 mm.

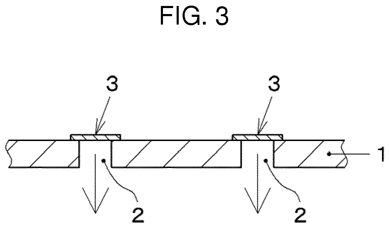

When a suctioning operation is performed by the vacuum pump, as shown in Fig. 3, the cell membrane 3 adsorbed to the face of the substrate 1 is suctioned from the holes 4b and 4c toward the center portion of each hole 2 and hence is pressed against the face of the substrate 1. The suction direction is schematically depicted by the arrow directed downward from each hole 2 of Fig. 3. The suction pressure is desirably set to 800 Pa or 1000 Pa as an example. By doing so, the cell membrane 3 can be continuously pressed against the face of the substrate 1 through the hole 2 while the cell membrane 3 is not damaged and does not pass through the hole 2.

The substrate placing member 4 is reversed while the suctioning operation is performed and the saucer 6 having the second buffer solution charged therein is fitted into the substrate placing member 4. At least the bottom face of the saucer 6 is formed of glass and the peripheral edge portion 6a is formed of polystyrene. The fitting of the saucer 6 is performed in such a manner that the peripheral edge portion 6a of the saucer 6 is fitted into the spot-facing portion and four points separated from one other at the same interval of 90° on the outside of the peripheral edge portion 6a are locked by a ball plunger 9 shown in Figs. 5A and 5B. Further, in order to first show the locking at the outside of the peripheral edge portion 6a by the ball plunger 9, the ball plunger 9 is shown in Figs. 4B and 5B on the assumption that the positions are different from the installation position of the ball plunger 9 of Figs. 4A and 5A. Further, the ball plunger 9 shown in appearance is omitted in Figs. 4B and 5B.

By the setting of the height dimensions of the abutting portions 4a and 4a of the substrate placing member 4, the thickness T of the substrate 1, and the amount of the second buffer solution charged into the saucer 6, the bottom face of the inner face of the saucer 6 contacts the placement face for placing the substrate 1 in the substrate placing member 4 when the saucer 6 is fitted into the substrate placing member 4 and, in addition, when the bottom face contacts the placement face, the cell membrane 3 placed on the face of the substrate 1 is sealed by the second buffer solution.

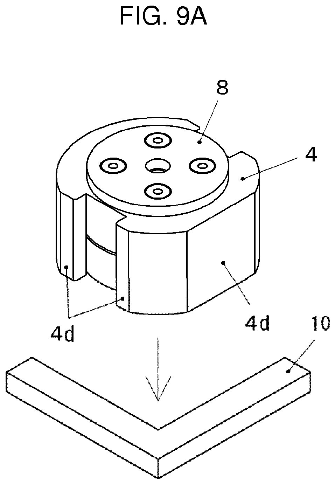

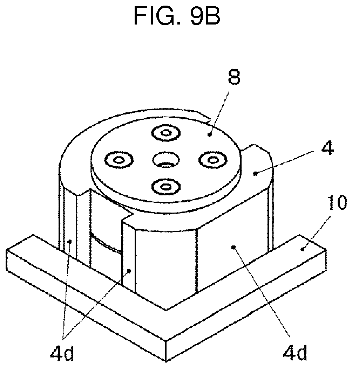

Next, the substrate placing member 4 is placed on a microscope while the suctioning operation is continued by the pump, and the cell membrane 3 is observed and analyzed. The substrate placing member 4 is placed on the microscope in such a manner that the restraining member 8 of the substrate placing member 4 is directed upward and the abutting portions 4d and 4d of two axial directions, which is provided in the outermost face of the substrate placing member 4 contact an L-shaped abutting unit 10 provided on a stage of the microscope while the suctioning operation is performed (see Fig. 9). Thus, the abutting unit 10 corresponds to a placement position in which the substrate placing member 4 is placed in the microscope. Each of the abutting portions 4d and 4d is provided in at least a part of the outermost face of the substrate placing member 4. In the invention, it is assumed that the microscope constitutes the cell membrane observation and analysis device. As the microscope, a fluorescence microscope, an electronic microscope, a scanning probe microscope (including an atomic force microscope), a confocal microscope, or a laser microscope may be used. The ball plunger 9 and the joint port 12 are not shown in Fig. 9.

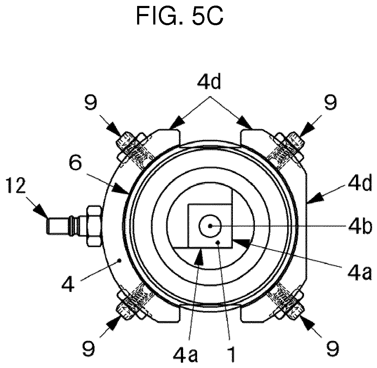

The abutting portions 4d and 4d are also provided in the directions parallel to two orthogonal axes similarly to the abutting portions 4a and 4a. Thus, as shown in Figs. 4C and 5C, the abutting portions 4a and 4a and the abutting portions 4d and 4d are provided in parallel. Thus, when the L-shaped abutting unit 10 is prepared on the stage of the microscope, two axial directions of the stage are respectively parallel to two axial directions of the hole of the substrate 1. Thus, the number of the cell membranes 3 can be easily measured when the cell membrane 3 is observed and analyzed.

Next, each cell membrane 3 pressed against the face of the substrate 1 by the suctioning of the pump is subjected to imaging observation and analysis using a fluorescence microscope or the like (hereinafter, referred to as "fluorescence observation and analysis" for convenience of description). The fluorescence observation and analysis according to the invention can be largely divided into two observation methods and analysis methods. The first observation method and analysis method is to observe and analyze the cell membrane 3 by attaching a fluorescent dyeing agent to the cell membrane 3 as a sample and emitting red or green light therefrom by irradiating excitation light to the fluorescent dyeing agent. The second observation method and analysis method is individually analyze the vital function of each face of the cell membrane 3 in each face.

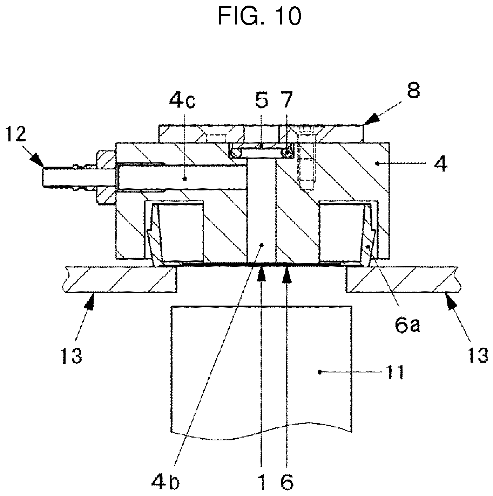

First, an observation method and an analysis method using a fluorescent dyeing agent will be described. Fig. 10 shows an observation and analysis state using an inverted microscope in which an objective lens 11 is provided at the downside of the drawing with a hole of a stage 13 of a microscope interposed therebetween. In the fluorescence observation and analysis using the inverted microscope, excitation light is incident upward from the downside through the objective lens 11 and the excitation light irradiates the fluorescent dyeing agent attached to the cell membrane 3 as the sample, thereby emitting red or green light. Accordingly, the appearance, the shape, or the outline of the entire cell membrane 3 can be observed and analyzed by fluorescence.

Further, in the visible light observation and analysis (the transmission observation and analysis) using the inverted microscope, a transmitted illumination unit (not shown) is attached to the stage 13. For this reason, the transmitted illumination unit can be brought to the upside of the cell membrane 3 during the visible light observation and analysis. Thus, when illumination light of a visible light region is irradiated downward from the upside of Fig. 10, the appearance, the shape, or the outline of the entire cell membrane 3 can be observed and analyzed.

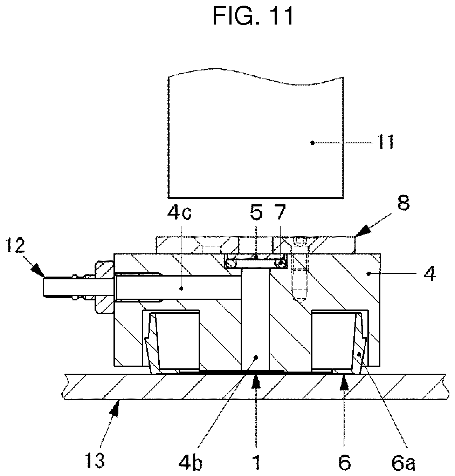

Further, the structure of the microscope is not limited to the inverted type, and the substrate placing member 4 may be placed on an upright microscope. Fig. 11 shows an observation and analysis state using an upright microscope in which the objective lens 11 is provided at the upside of Fig. 11.

In the case of the observation and the analysis using the upright microscope, the objective lens is located at the upside of Fig. 11 and hence the observation and the analysis are performed from the upside of the substrate 1. In the fluorescence observation and analysis, excitation light is incident downward from the upside through the objective lens 11 and the excitation light is irradiated to the fluorescent dyeing agent attached to the cell membrane 3 as the sample so that red or green light is emitted. Thus, the appearance, the shape, or the outline of the entire cell membrane 3 can be observed and analyzed by fluorescence.

Further, in the visible light observation and analysis (the transmission observation and analysis), the transmitted illumination unit (not shown) is attached to the stage 13. For this reason, the transmitted illumination unit can be brought to the vicinity of the downside of the cell membrane 3 during the visible light observation and analysis. Thus, when illumination light of a visible light region is irradiated upward from the downside of Fig. 11, the appearance, the shape, or the outline of the entire cell membrane 3 can be observed and analyzed.

Alternatively, in the upright microscope, the appearance, the shape, or the outline of the entire cell membrane 3 may be observed and analyzed by visible light while the substrate placing member 4 is turned over so that the placement side for the substrate 1 faces the objective lens 11. In this case, the saucer 6 is not used. Since the placement side for the substrate 1 faces the objective lens 11, the cell membrane 3 and the objective lens 11 can be closer to each other and hence the cell membrane 3 can be observed and analyzed with higher magnification.

Next, the method of individually observing and analyzing the vital function of each face of the cell membrane 3 will be described. The cell membrane 3 placed on each hole 2 keeps the front and rear faces of the sampled cell membrane. For that reason, it is possible to independently evaluate the binding property of target molecules such as protein or drug toward the front and rear faces of the cell membrane 3. For example, it is possible to estimate the affinity of both faces of the cell membrane 3 of the target molecule by analyzing the concentration-dependent binding amount of the fluorescence labeled target molecules bound to the face of the cell membrane 3. Thus, in the invention, it is possible to individually analyze the vital function of each face of the cell membrane 3. In the fluorescence observation and analysis using the inverted microscope shown in Fig. 10, it is possible to estimate the affinity toward the face near the objective lens 11 among both faces of the cell membrane 3 of the target molecule in such a manner that the fluorescence labeled target molecules are bound to the face of the cell membrane 3 and excitation light is incident upward from the downside through the objective lens 11.

Further, in the fluorescence observation and analysis using the upright microscope shown in Fig. 11, it is possible to estimate the affinity toward the face opposite to the objective lens 11 among both faces of the cell membrane 3 of the target molecule in such a manner that the fluorescence labeled target molecules are bound to the face of the cell membrane 3 and excitation light is incident downward from the upside through the objective lens 11.

In the case where the substrate 1 is formed of a material of a transparent body when the cell membrane 3 is subjected to the visible light observation and analysis, the appearance, the shape, or the outline of the entire cell membrane 3 can be accurately observed and analyzed. Meanwhile, in a case where the substrate 1 is formed of plastic or silicon having no light-transmissivity in a visible light region or has no light transmissivity by attaching a shielding film or a seal to the front face of the substrate 1, it is possible to observe and analyze only the same areas as the opening area of the hole 2 in the cell membranes when the cell membrane 3 is subjected to the visible light observation and analysis. However, since the amount of light transmitted through the substrate 1 becomes a constant value in response to the entire opening area of the holes 2, the quantitative observation or analysis can be realized.

As described above, the cell membrane observation and analysis device and the cell membrane observation and analysis method according to the invention can highly sensitively observe and analyze a vital function of a cell membrane derived from a cancer cell of cells such as a human cell or an animal cell like a mammalian cell other than a human cell and an insect cell.

The cell membrane 3 can be observed and analyzed from both faces. Thus, since it is possible to highly sensitively track the vital function occurring in the cell membrane 3, there is no need to prepare a large number of cell samples for the drug sensitivity test and the test can be conducted rapidly. Moreover, the effort of exchanging the samples is saved and both faces of the cell membrane 3 can be observed and analyzed rapidly. Further, a desired vital reaction can be generated in both faces or an arbitrary one face of the cell membrane 3.

Even when the second buffer solution or the buffer solution (the suspension) having the cell membrane 3 dispersed therein is suctioned by a pump so that the solution contacts the light transmission window 5 through the hole 4b and the light transmission window 5 gets dirty, the light transmission window 5 can be easily cleaned since the light transmission window 5 can be detachable from the substrate placing member 4.

### Examples

Hereinafter, examples of the invention will be described, but the invention is not limited to the examples below.

A cell membrane observation and analysis device according to the embodiment uses an inverted microscope and includes a substrate formed of a transparent body. A substrate formed of quartz glass and having an external dimension with a length of 10 mm, a width of 10 mm, and a thickness of 0.2 mm was prepared so as to have a property of light transmissivity of 90% or more and a refractive index of 1.5 with respect to visible light having a wavelength λ of 588 nm. A hole was formed in the substrate so as to have a circular shape as a planar shape and a straight hole shape as a cross-sectional shape. A total of 625 holes were formed as 25 units by 25 lines. The diameter of the hole was 2.0 μm, the gap between the center portions of the holes was 40 μm, and the aspect ratio of each hole was 100.

As a target cell membrane for observation and analysis, a cell derived from a HeLa cell was used and a cell incubated in a MEM medium was used. A HeLa cell reaching about 90% of confluency was prepared in a dish of 10 cm. As a sample including the HeLa cell, a sample including 500,000 cells/ml was prepared. Further, 3, 3'-dioctadecyloxacarbocyanine perchloric acid salt (DiO) (1 mg/ml) of 10 μl was added to MEM of 5 ml and then was incubated for 10 minutes at the condition of 37°C and 5% of CO₂.

Next, the HeLa cell was cleaned by a buffer solution A (having 2 mM of CaCl₂, 10 mM of Tris/HCl of pH 7.5, and 150 mM of NaCl) of 5 ml. Moreover, a buffer solution B (obtained by 25 mM of formaldehyde and 2 mM of DTT to the buffer solution A) was added to the HeLa cell.

Next, the solution was incubated for 30 minutes at the condition of 37°C and 5% of CO₂, supernatant thereof was dyed by a fluorescent dyeing agent, and a GPMVs (giant plasma membrane vesicles) suspension was obtained. The GPMVs has a spherical cell membrane structure of about 10 μm cut out from a cultured cell.

The GPMVs suspension (500,000 units/ml) of 50 μl was dripped onto the substrate and was left for 1 minute at a reduced pressure of at least 20% of the atmospheric pressure. As a substrate placing member, a light transmission window, a light transmission window restraining member, and a saucer, the components mentioned in the above-described embodiment were used and the arrangement or the assembly of the components was set as in the above-described embodiment.

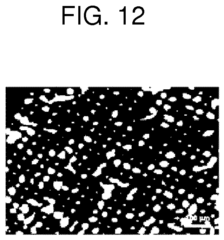

Further, a vacuum pump was used for the suctioning of GPMVs and a suction pressure was set to 800 Pa. While a suctioning operation is continued by a vacuum pump, the substrate placing member was placed on an inverted microscope, a dyeing treatment was performed by a fluorescent dyeing agent, and light of an infrared light or visible light region was irradiated to the substrate, thereby performing fluorescence observation and analysis. In this way, the HeLa cell membrane was observed and analyzed. The fluorescence microscope picture is shown in Fig. 12. As described above, the object of the invention is achieved.

### Explanation on reference numerals

- 1:: Substrate
- 2:: Hole
- 3:: Cell membrane
- 4:: Substrate placing member
- 4a,: 4d: Abutting portion
- 4b,: 4c: Hole
- 5:: Light transmission window
- 6:: Saucer
- 6a:: Peripheral edge portion of saucer
- 7:: Ring member
- 8:: Light transmission window restraining member
- 9:: Ball plunger
- 10:: Abutting unit
- 11:: Objective lens of microscope
- 12:: Joint port toward pump
- 13:: Stage

## Claims

1. A cell membrane observation and analysis device comprising at least:
a microscope; and
a substrate which has a plurality of holes,
wherein a cell membrane is placed on each hole of the substrate so that the cell membrane is placed on the substrate, the cell membrane is immersed into a buffer solution while the cell membrane is suctioned through the hole, and, further, the buffer solution is sealed, thereby observing and analyzing the cell membrane.

2. The cell membrane observation and analysis device according to claim 1, further comprising:
a substrate placing member that places the substrate thereon,
wherein abutting portions of two orthogonal axial directions are provided in at least a part of an outermost face of the substrate placing member and in a substrate placement portion for placing the substrate, and each of them is provided in parallel with two axes.

3. The cell membrane observation and analysis device according to claim 2, further comprising:
a light transmission window through which light is transmitted and which is provided at the other side of a substrate installation side in the substrate placing member,
wherein the cell membrane is irradiated with light transmitted through the light transmission window, and
wherein the light transmission window is detachable from the substrate placing member.

4. The cell membrane observation and analysis device according to any one of claims 1 to 3,
wherein a vital function of each of faces of the cell membrane is individually analyzed in each face.

5. The cell membrane observation and analysis device according to claim 3 or 4,
wherein the light transmission window is formed of glass having light transmissivity of 90% or more with respect to visible light.

6. A cell membrane observation and analysis method comprising:
preparing a substrate having a plurality of holes;
preparing a suspension by dispersing a cell membrane in a buffer solution;
dripping the suspension onto a face of the substrate;
suctioning the cell membrane through each hole by a pump so that the cell membrane is placed on each hole;
immersing the cell membrane into a second buffer solution while continuously suctioning the cell membrane;
sealing the second buffer solution; and
observing and analyzing the cell membrane by a microscope.

7. The cell membrane observation and analysis method according to claim 6, comprising:
further preparing a substrate placing member having a substrate placed thereon, the substrate placing member having abutting portions of two orthogonal axial directions provided in at least a part of an outermost face of the substrate placing member and in a substrate placement portion for placing the substrate and each of them being provided in parallel with two axes;
bringing two orthogonal edges of the outermost face of the substrate into contact with the abutting portions of the substrate placement portion so that the substrate is placed on the substrate placement portion of the substrate placing member; and
bringing the abutting portions of two orthogonal axial directions in the outermost face of the substrate placing member into contact with abutting unit of two orthogonal axial directions in a substrate placing member placement position of the microscope so that the substrate placing member is placed on the substrate placing member placement position of the microscope.

8. The cell membrane observation and analysis method according to claim 6 or 7,
wherein a vital function of each of faces of the cell membrane is individually analyzed in each face.
